# EUROPEAN PATENT SPECIFICATION

(11) **EP 0 569 537 B1**
(45) Date of publication and mention of the grant of the patent: **17.06.1998**
(21) Application number: 92906219.8
(22) Date of filing: 30.01.1992
(51) Int. Cl.: C12P 21/08, C12N 5/20, G01N 33/53

(54) **MONOCLONAL ANTIBODIES TO PUTATIVE HCV ENVELOPE REGION AND METHODS FOR USING SAME**
MONOKLONALE ANTIKÖRPER GEGEN MÖGLICHE HCV-HÜLLREGIONEN UND METHODEN DIESE ZU BENUTZEN
ANTICORPS MONOCLONAUX CONTRE LES REGIONS D'ENVELOPPE PUTATIVES DU VIRUS VHC ET PROCEDES D'UTILISATION DE CEUX-CI

(30) Priority: 31.01.1991 US 648475
(43) Date of publication of application: 18.11.1993
(73) Proprietor: ABBOTT LABORATORIES, Abbott Park, Illinois 60064-3500 (US)
(72) Inventor: GIBADLO, Mary, S., Arlington Heights, IL 60005-2746 (US); TYNER, Joan, D., Beach Park, IL 60087-2208 (US); MIMMS, Larry, T., Lake Villa, IL 60046 (US); VALLARI, David, S., Grayslake, IL 60030 (US)
(74) Representative: Modiano, Guido, Dr.-Ing.
(86) International application number: US9200687
(87) International publication number: WO9213892

(56) References cited:
- EP-A- 0 318 216
- EP-A- 0 445 423
- WO-A-82/02774
- WO-A-90/11089
- WO-A-91/15574
- MODERN APPROACHES TO NEW VACCINES INCLUDING PREVENTION OF AIDS.9TH ANNUAL MEETING,COLD SPRING HARBOR September 1991 , NEW YORK,USA MATSUURA ET AL 'GLYCOSYLATED ENVELOPE PROTEIN OF HEPTITIS C VIRUS EXPRESSED IN ANIMAL CELLS' & VACCINES 92 vol. 0, no. 0 , 1992 pages 309 - 314
- 42ND ANNUAL MEETING OF THE AMERICAN ASSOCATION FOR THE STUDY OF LIVER DISEASES November 1991 , CHICAGO,ILL,USA GOESER ET AL 'SCREENING FOR ANTIBODIES TO DISTINCT PEPTIDES FROM THE PUTATIVE C- AND ENV- REGION OF THE HEPATITIS C VIRUS' & HEPATOLOGY vol. 14, no. 4(2) , 1991 page 118A
- 42ND ANNUAL MEETING OF THE AMERICAN ASSOCATION FOR THE STUDY OF LIVER DISEASES November 1991 , CHICAGO,ILL,USA TIBBS ET AL 'DETECTION OF ANTIBODIES TO CORE,ENVELOPE,NS-1,NS-3 AND NS-5 REGIONS OF HEPATITIS C VIRUS IN PATIENTS WITH CHRONIC VIRAL HEPATITIS' & HEPATOLOGY vol. 14, no. 4(2) , 1991 page 68A
- 42ND ANNUAL MEETING OF THE AMERICAN ASSOCIATION FOR THE STUDY OF LIVER DISEAESE November 1991 , CHICAGO,ILL,USA HIRAMATSU ET AL 'DETECTION OF HEPATITIS C VIRUS (HCV) INFECTED HEPATOCYTES IN CHRONIC LIVER DISEASE' & HEPATOLOGY vol. 14, no. 4(2) , 1991 page 66A
- JAPANESE JOURNAL OF EXPERIMENTAL MEDICINE, vol. 60, no. 4, 1990; H. OKAMOTO et al., pp. 223-233/
- JAPANESE JOURNAL OF CANCER RESEARCH, vol. 81, November 1990; N. KATO et al., pp. 1092-1094/
- NUCLEIC ACIDS RESEARCH, vol. 18, no. 15, 1990; K. TAKEUCHI et al., p. 4626/

## Description

### Background of the Invention

This invention relates generally to antibodies which specifically bind to hepatitis C virus (HCV), and more specifically, relates to a panel of novel hybridoma cells lines which secrete monoclonal antibodies to putative HCV envelope regions, and methods for using these monoclonal antibodies.

Descriptions of hepatitis diseases causing jaundice and icterus have been known to man since antiquity. Viral hepatitis is now known to include a group of viral agents with distinctive viral organization protein structure and mode of replication, causing hepatitis with different degrees of severity of hepatic damage through different routes of transmission. Acute viral hepatitis is clinically diagnosed by well-defined patient symptoms including jaundice hepatic tenderness and an elevated level of liver transaminases such as aspartate transaminase and alanine transaminase.

Serological assays currently are employed to further distinguish between hepatitis-A and hepatitis-B. Non-A non-B Hepatitis (NANBH) is a term first used in 1975 that described cases of post-transfusion hepatitis not caused by either hepatitis A virus or hepatitis B virus. Feinstone et al., New Engl. J. Med. 292:454A57 (1975). The diagnosis of NANBH has been made primarily by means of exclusion on the basis of serological analysis for the presence of hepatitis A and hepatitis B. NANBH is responsible for about 90% of the cases of posttransfusion hepatitis. Hollinger et al. in N. R. Rose et al., eds., Manual of Clinical Immunology, American Society for Microbiology, Washington, D. C., 558-572 (1986).

Attempts to identify the NANBH virus by virtue of genomic similarity to one of the known hepatitis viruses have failed thus far, suggesting that NANBH virus has a distinctive genomic organization and structure. Fowler et al., J. Med. Virol. 12:205-213 (1983), and Weiner et al., J. Med. Virol. 21:239-247 (1987). Progress in developing assays to detect antibodies specific for NANBH has been hampered by difficulties encountered in identifying antigens associated with the virus. Wards et al., U. S. Patent No. 4,870,076; Wards et al.. Proc. Natl. Acad. Sci. 83:6608-6612 (1986); Ohori et al., J. Med. Virol. 12:161-178 (1983); Bradly et al., Proc. Natl. Acad. Sci. 84:6277-6281 (1987), Akatsuka et al., J. Med. Virol. 20:43-56 (1986).

In May of 1988, a collaborative effort of Chiron Corporation with the Centers for Disease Control resulted in the identification of a putative NANB agent. hepatitis C virus (HCV). M. Houghton et al. cloned and expressed in E. coli a NANB agent obtained from the infectious plasma of a chimp. Kuo et al., Science 244:359-361 (1989); Choo et al., Science 244:362-364 (1989). cDNA (copy DNA) sequences from HCV were identified which encode antigens that react immunologically with antibodies present in a majority of the patients clinically diagnosed with NANBH. Based on the information available and on the molecular structure of HCV, the genetic makeup of the virus consists of single stranded linear RNA (positive strand) of molecular weight approximately 9.5 kb, and possessing one continuous translational open reading frame. J. A. Cuthbert, Amer. J. Med. Sci. 299:346-355 (1990). It is a small enveloped virus resembling the Flaviviruses. Investigators have made attempts to identify the NANB agent by ultrastructural changes in hepatocytes in infected individuals. H. Gupta, Liver 8:111-115 (1988); D.W. Bradly J. Virol. Methods 10:307-319 (1985). Similar ultrastructural changes in hepatocytes as well as PCR amplified HCV RNA sequences have been detected in NANBH patients as well as in chimps experimentally infected with infectious HCV plasma. T. Shimizu et al., Proc. Natl. Acad. Sci. 87:6441-6444 (1990).

Considerable serological evidence has been found to implicate HCV as the etiological agent for post-transfusion NANBH. H. Alter et al., N. Eng. J. Med. 321:1494-1500 (1989); Estaben et al., The Lancet: Aug. 5:294-296 (1989); C. Van Der Poel et al., The Lancet Aug. 5:297-298 (1989); G. Sbolli, J. Med. Virol. 30:230-232 (1990); M. Makris et al., The Lancet 335:1117-1119 (1990). Although the detection of HCV antibodies eliminates 70 to 80% of NANBH infected blood from the blood supply system, the antibodies apparently are readily detected during the chronic state of the disease, while only 60% of the samples from the acute NANBH stage are HCV antibody positive. H. Alter et al., New Eng. J. Med. 321:1994-1500 (1989). The prolonged interval between exposure to HCV and antibody detection, and the lack of adequate information regarding the profile of immune response to various structural and non-structural proteins raises questions regarding the infectious state of the patient in the antibody negative phase during NANBH infection. Therefore, there is a need for the development of assay systems to identity acute infection to HCV and the presence of HCV.

As further background to the present invention, M. Houghton et al. in WO-A-90/11089 and EP-A-O 318 216 disclose HCV peptides, including peptides containing epitopes belonging to the envelope region, useful in HCV diagnostics and vaccines. Monoclonal antibodies to such peptides are disclosed.

WO-A-91/15574, which is prior art under the terms of Article 54(3) and (4) EPC, discloses antibodies raised against the HCV envelope region.

WO 82/02774 discloses separated HCV envelope antigens for use as immunogens to prepare antibodies.

### Summary of the Invention

The present invention provides a panel of highly specific and novel monoclonal antibodies that can be employed for the detection of putative HCV envelope regions. The monoclonal antibodies specifically bind to peptides derived from the putative HCV envelope (ENV) gene. The hybridomas which secrete these monodonal antibodies are identified as follows: Hybridoma cell line 16-407-209 (A.T.C.C. deposit No. HB 10601, secreting monoclonal antibody 16-407-209), and hybridoma cell line 16-803-174 (A.T.C.C. deposit No. HB 10605, secreting monoclonal antibody 16-803-174). The specificity of these monoclonal antibodies enables the advantageous identification of putative HCV envelope region, which identification can be useful in differentiation studies as well as in the diagnosis and evaluation of HCV (NANB) infections.

In a preferred assay format, a test sample which may contain HCV antigens is contacted with a solid phase to which a monoclonal anti-HCV envelope region antibody of the invention or a fragment thereof has been bound, to form a mixture. This mixture is incubated for a time and under conditions sufficient for antigen/antibody complexes to form. The so-formed complexes then are contacted with an indicator reagent comprising a monoclonal antibody of the invention or a fragment thereof, specific for the HCV antigen which is attached to a signal generating compound, to form a second mixture. This second mixture is reacted for a time and under conditions sufficient to form antibody/antigen/antibody complexes. The presence of HCV antigen is determined by detecting the measurable signal generated. The amount of HCV present in the test sample, thus the amount of HCV antigen captured on the solid phase, is proportional to the amount of signal generated.

Alternatively, an indicator reagent comprising a monoclonal antibody of the invention, or fragment thereof, specific for HCV envelope region and a signal generating compound is added to a monoclonal anti-HCV antibody of the invention or fragment thereof coated on a solid phase and the test sample, to form a mixture. This mixture is incubated for a time and under conditions sufficient to form antibody/antigen/antibody complexes. The presence and amount of HCV present in the test sample, and thus the amount of HCV antigen captured on the solid phase, is determined by detecting the measurable signal. The amount of HCV present in the test sample is proportional to the amount of signal generated.

In another alternate assay format, one or a combination of more than one monoclonal antibody of the invention can be employed as a competitive probe for the detection of antibodies to putative HCV envelope region. For example, HCV envelope region proteins, either alone or in combination, can be coated on a solid phase. A test sample suspected of containing antibody to HCV envelope region then is incubated with an indicator reagent comprising a signal generating compound and a monodonal antibody of the invention for a time and under conditions sufficient to form antigen/antibody complexes of either the test sample and indicator reagent to the solid phase or the indicator reagent to the solid phase. The reduction in binding of the monoclonal antibody to the solid phase can be quantitatively measured. A measurable reduction in the signal compared to the signal generated from a confirmed negative NANBH test sample would indicate the presence of anti-HCV envelope antibody in the test sample.

In yet another assay format, a test sample is contacted with a solid phase to which HCV proteins are attached and an indicator reagent comprising a monoclonal antibody of the invention or fragment thereof specific for HCV attached to a signal generating compound, to form a mixture. The mixture is incubated for a time and under conditions sufficient for antibody/antigen complexes to form. The presence of anti-HCV antibody present in the test sample is determined by detecting the measurable signal generated, and comparing the signal to the measured signal generated from a known negative sample. A measurable reduction of signal of the test sample, compared to the known negative sample's signal, is indicative of the presence of anti-HCV antibodies. Competitive assays for the detection of anti-HCV antibody using antigens free in solution also can be performed.

The presence of putative HCV envelope region can be detected in a tissue sample by contacting the tissue sample with an indicator reagent comprising a signal generating compound attached to a monoclonal antibody of the invention which specifically binds to HCV envelope region or fragment thereof, to form a mixture. This mixture is incubated for a time and under conditions sufficient for antigen/antibody complex to form. The presence of HCV envelope region present in the tissue sample is determined by detecting the signal generated.

Also provided are kits for using the monoclonal antibodies of the invention. In the following description, reference is made to CKS and SOD fusion polypeptides. CKS (E. coli CMP-KDO synthetase) is known from EP-A-O 331 961 which was published on September 13, 1989. SOD (superoxide dismutase) is known from EP-A-O 445 423 which was published on September 11, 1991.

### Brief Description of the Drawings

FIG. 1 is a map of the HCV GENOME representing the non-structural (NS) genes and the structural genes, core (C) and envelope (E).

FIGS. 2 to 13 are photographs of Westem blots showing the reactivity of the monoclonal antibodies of the invention, where
lanes 1 to 3 contain monoclonal antibodies against HCV 33C protein (6-296-534 in lane 1, 6-914-518 in lane 2 and 6-1070-110 in lane 3);
lanes 4-6 contain monoclonal antibodies against HCV CORE (13-975-157 in lane 4, 14-153-234 in lane 5 and 14-1350-210 in lane 6);
lanes 7 and 8 contain monoclonal antibodies against the putative HCV ENV region (16-407-209 in lane 7 and 16-803-174 in lane 8);
lanes 9-10 contain monoclonal antibodies against HCV C-100 (25-1518-105 in lane 9, 28-735-355 in lane 10);
line 11 contains monoclonal antibody against CKS (29-121-236 in lane 11);
lane 12 contains a normal mouse serum control; and
lane 13 contains a negative control of antibody diluent.

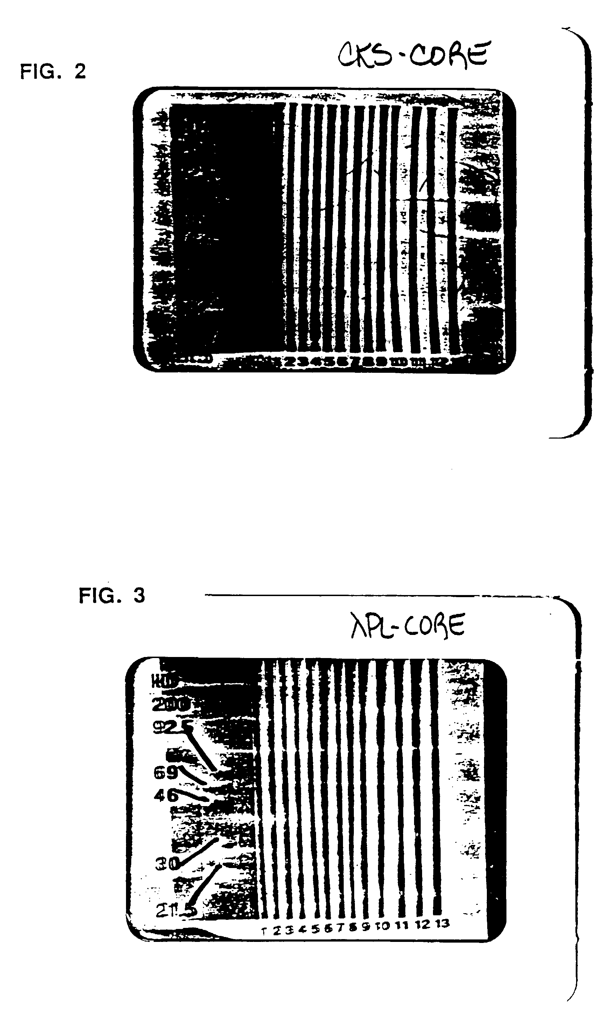

FIG. 2 is an electroblot of these monoclonal antibodies run against CKS-CORE;
FIG. 3 is an electroblot of these monoclonal antibodies run against λPL-CORE;

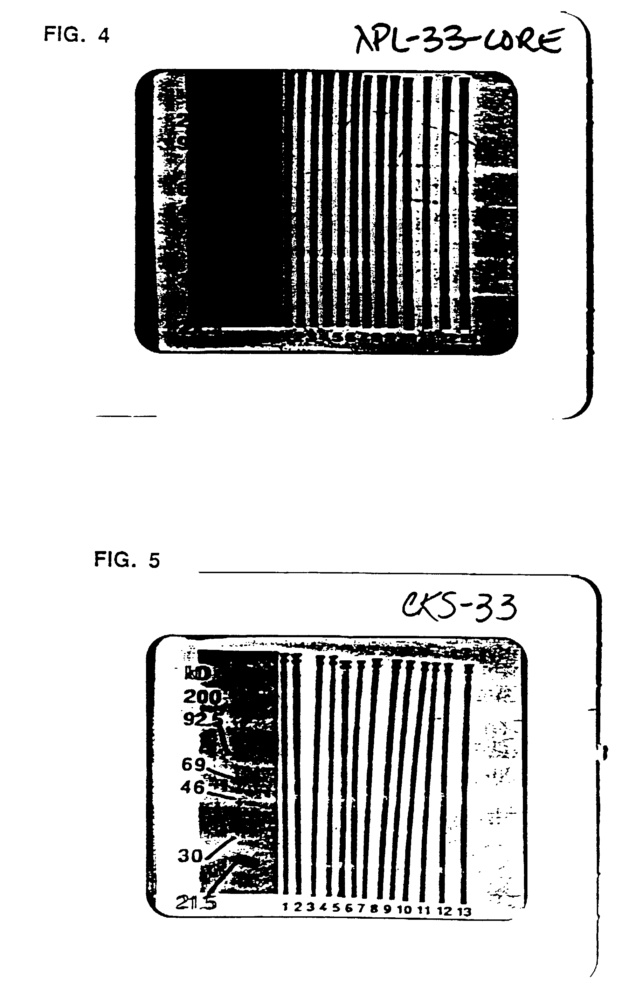

FIG. 4 is an electroblot of these monoclonal antibodies run against λPL-33C-CORE;
FIG. 5 is an electroblot of these monoclonal antibodies run against CKS-33C;

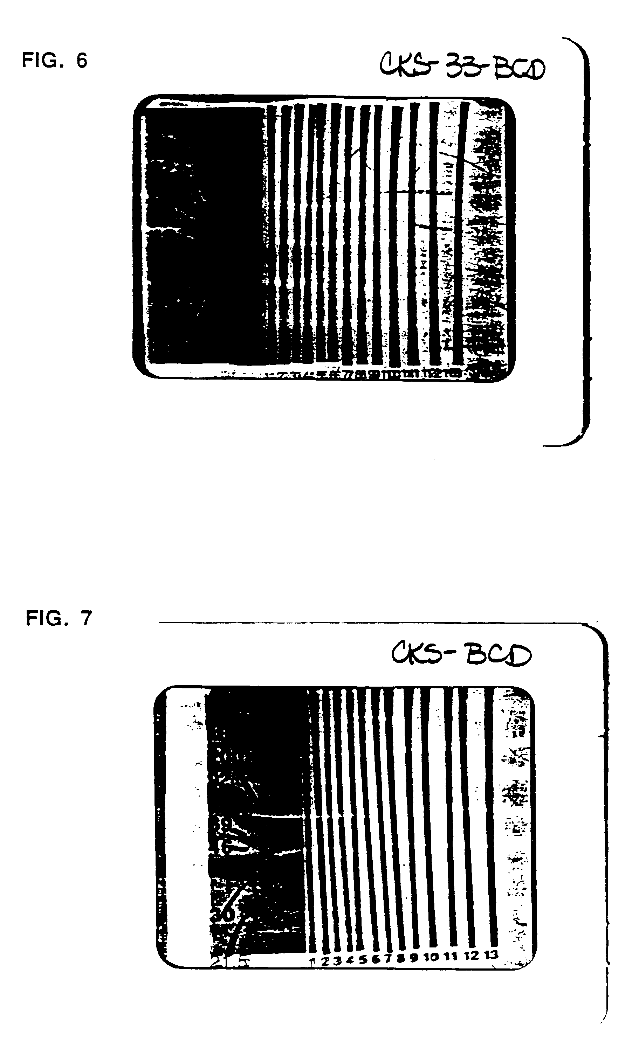

FIG. 6 is an electroblot of these monoclonal antibodies run against CKS-33C-BCD:
FIG. 7 is an electroblot of these monoclonal antibodies run against CKS-BCD;

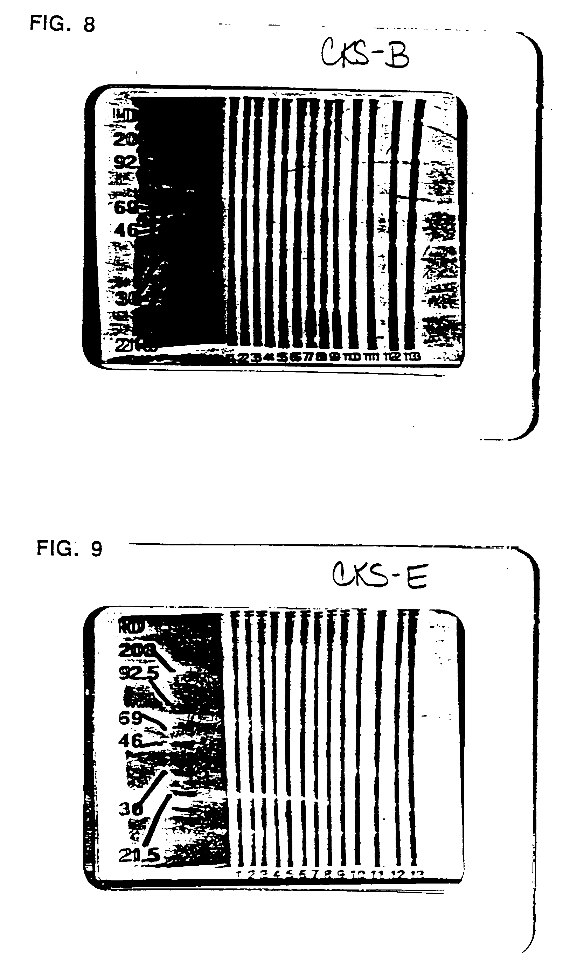

FIG. 8 is an electroblot of these monoclonal antibodies run against CKS-B;
FIG. 9 is an electroblot of these monoclonal antibodies run against CKS-E;

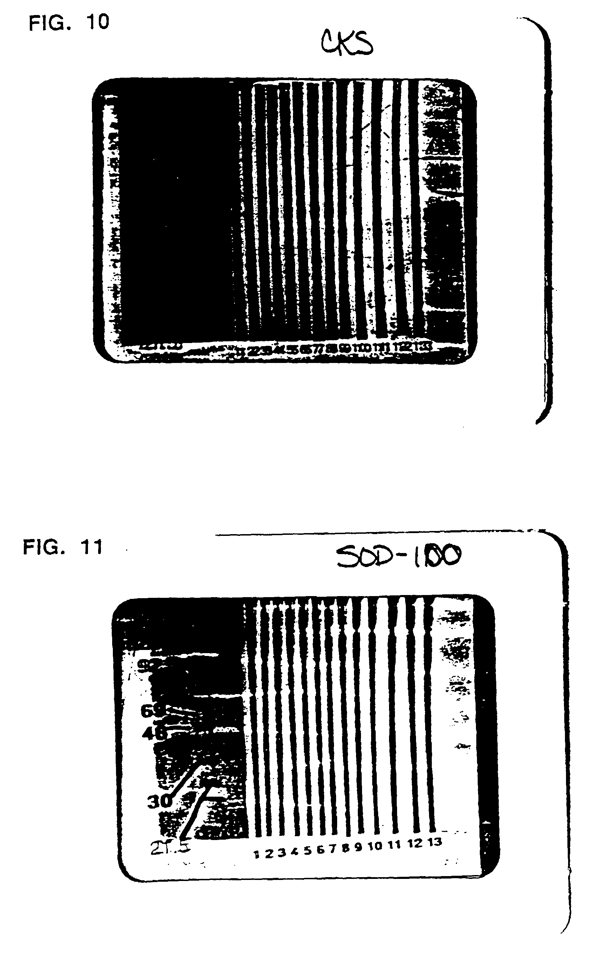

FIG. 10 is an electroblot of these monoclonal antibodies run against CKS;

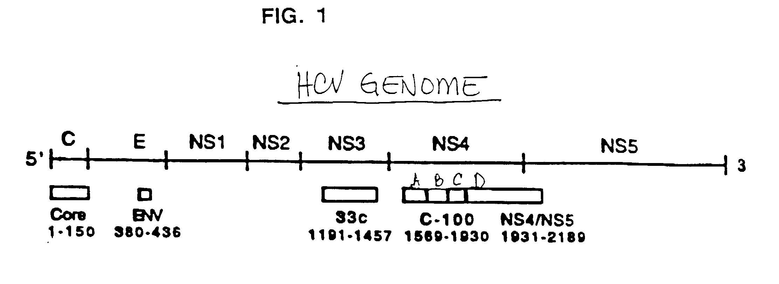

FIG. 1 is an electroblot of these monoclonal antibodies run against SOD-100;

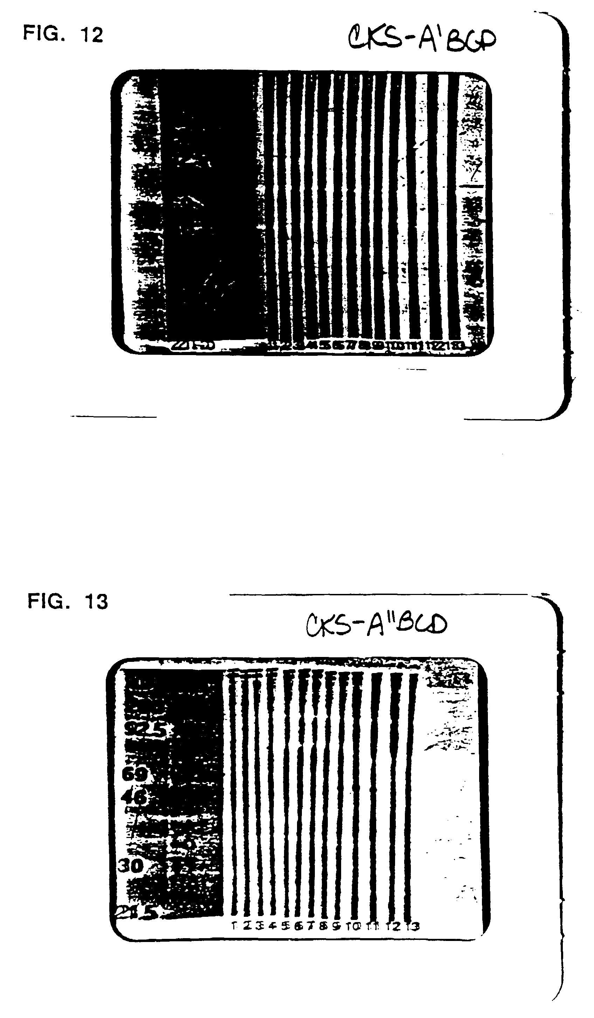

FIG. 12 is an electroblot of these monodonal antibodies run against CKS-A'BCD; and
FIG. 13 is an electroblot of these monoclonal antibodies run against CKS-A"BCD.

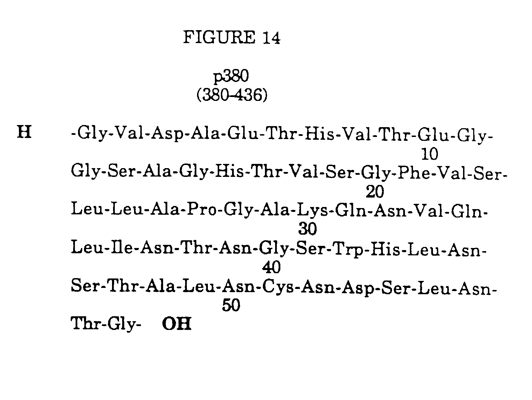

FIG. 14 is the amino acid sequence of the putative ENV domain of the HCV genome 380-436.

### Detailed Description of the Invention

The present invention provides novel monoclonal antibodies to putative HCV envelope region, methods for using the monoclonal antibodies, and kits which contain these monoclonal antibodies.

The monodonal antibodies of the present invention can be employed in various assay systems to determine the presence, if any, of putative HCV envelope region proteins in a test sample. Fragments of these monoclonal antibodies provided also may be used. For example. in a first assay format, a monoclonal anti-HCV envelope region antibody or fragment thereof, or a combination of these antibodies, which has been coated on a solid phase, is contacted with a test sample which may contain putative HCV envelope region proteins, to form a mixture. This mixture is incubated for a time and under conditions sufficient to form antigen/antibody complexes. Then, an indicator reagent comprising a monoclonal antibody of the invention or a fragment thereof, which specifically binds to the putative HCV envelope region, or a combination of these antibodies, to which a signal generating compound has been attached, is contacted with the antigen/antibody complexes to form a second mixture. This second mixture then is incubated for a time and under conditions sufficient to form antibody/antigen/antibody complexes. The presence of putative HCV envelope region present in the test sample and captured on the solid phase, if any, is determined by detecting the measurable signal generated by the signal generating compound. The amount of putative HCV envelope region present in the test sample is proportional to the signal generated.

Alternatively, a monoclonal anti-HCV envelope region antibody of the invention or fragment thereof, or a combination of these antibodies which is bound to a solid support, the test sample and an indicator reagent comprising a monoclonal antibody of the invention or fragments thereof, which specifically binds to putative HCV envelope region, or a combination of these antibodies to which a signal generating compound is attached, are contacted to form a mixture. This mixture is incubated for a time and under conditions sufficient to form antibody/antigen/antibody complexes. The presence, if any, of putative HCV envelope region proteins present in the test sample and captured on the solid phase is determined by detecting the measurable signal generated by the signal generating compound. The amount of HCV proteins present in the test sample is proportional to the signal generated.

In another alternate assay format, one or a combination of one or more monoclonal antibodies of the invention can be employed as a competitive probe for the detection of antibodies to putative HCV envelope region. For example, putative HCV envelope region proteins, either alone or in combination, can be coated on a solid phase. A test sample suspected of containing antibody to putative HCV envelope region then is incubated with an indicator reagent comprising a signal generating compound and at least one monoclonal antibody of the invention for a time and under conditions sufficient to form antigen/antibody complexes of either the test sample and indicator reagent to the solid phase or the indicator reagent to the solid phase. The reduction in binding of the monoclonal antibody to the solid phase can be quantitatively measured. A measurable reduction in the signal compared to the signal generated from a confirmed negative NANBH test sample indicates the presence of anti-HCV envelope antibody in the test sample.

In yet another detection method, each of the monoclonal antibodies of the present invention can be employed in the detection of HCV antigens in fixed tissue sections, as well as fixed cells by immunohistochemical analysis.

In addition, these monocional antibodies can be bound to matrices similar to CNBr-activated Sepharose and used for the affinity purification of specific HCV proteins from cell cultures, or biological tissues such-as blood and liver.

The monoclonal antibodies of the invention can also be used for the generation of chimeric antibodies for therapeutic use, or other similar applications.

The monoclonal antibodies or fragments thereof of the invention can be provided individually to detect putative HCV envelope region. Combinations of the monoclonal antibodies (and fragments thereof) provided herein also may be used together as components in a mixture or "cocktail" of anti-HCV envelope region antibodies with antibodies to other HCV regions, each having different binding specificities. Thus, this cocktail can include both the monoclonal antibodies of the invention which are directed to putative HCV envelope region proteins and other monoclonal antibodies to other antigenic determinants of the HCV genome.

The polyclonal antibody or fragment thereof which can be used with monoclonal antibodies of the invention in the assay formats should specifically bind to putative HCV envelope region or other HCV proteins used in the assay, such as HCV C-100 protein, HCV 33C protein or HCV CORE protein. The polyclonal antibody used preferably is of mammalian origin; human, goat, rabbit or sheep anti-HCV polyclonal antibody can be used. Most preferably, the polyclonal antibody is rabbit polyclonal anti-HCV antibody. The polyclonal antibodies used in the assays with monoclonal antibodies of the invention can be used either alone or as a cocktail of polyclonal antibodies. Since the cocktails used in the assay formats are comprised of monoclonal antibodies of the invention and of either monoclonal antibodies or polyclonal antibodies having different HCV specificity, they would be useful for diagnosis, evaluation and possibly for the prognosis of HCV infection, as well as for studying HCV protein differentation and specificity.

Test samples which can be tested by the methods of the present invention described herein include human and animal body fluids such as whole blood. serum, plasma, cerebrospinal fluid, urine, biological fluids such as cell culture supematants, fixed tissue specimens and fixed cell specimens. Solid supports are known to those in the art and include the walls of wells of a reaction tray, test tubes, polystyrene beads, magnetic beads, nitrocellulose strips, membranes, microparticles such as latex particles, and others.

The indicator reagent comprises a signal generating compound (label) which is capable of generating a measurable signal detectable by extemal means conjugated (attached) to a specific binding member for HCV. "Specific binding member" as used herein means a member of a specific binding pair. That is, two different molecules where one of the molecules through chemical or physical means specifically binds to the second molecule. In addition to being an antibody member of a specific binding pair for HCV, the indicator reagent also can be a member of any specific binding pair, including either hapten-anti-hapten systems such as biotin or anti-biotin, avidin or biotin, a carbohydrate or a lectin, a complementary nucleotide sequence, an effector or a receptor molecule, an enzyme cofactor and an enzyme, an enzyme inhibitor or an enzyme, and the like. An immunoreactive specific binding member can be an antibody, an antigen, or an antibody/antigen complex that is capable of binding either to HCV as in a sandwich assay, to the capture reagent as in a competitive assay, or to the ancillary specific binding member as in an indirect assay.

The various signal generating compounds (labels) contemplated include chromogens, catalysts such as enzymes, luminescent compounds such as fluorescein and rhodamine, chemiluminescent compounds, radioactive elements, and direct visual labels. Examples of enzymes include alkaline phosphatase, horseradish peroxidase, beta-galactosidase, and the like. The selection of a particular label is not critical, but it will be capable of producing a signal either by itself or in conjunction with one or more additional substances.

It is contemplated that the reagent employed for the assay can be provided in the form of a kit with one or more containers such as vials or bottles, with each container containing a separate reagent such as a monoclonal antibody, or a cocktail of monodonal antibodies, employed in the assay.

### Materials and Methods

### Production of recombinant HCV antigens and immunogens

Synthetic peptides corresponding to regions within the putative ENV domain of the HCV genome were made by automated peptide synthesizer. The following peptides were constructed utilizing standard methods known in the art:
- ENV: 380-436
405-436.

ENV 380-436 is described in EP-A-0 445 423 which was published September 11, 1991.

FIG. 1 is a map of the HCV genome and the approximate locations of HCV regions. The amino acid sequence for the putative ENV domain of the HCV genome (p380-436) is shown in FIG. 14.

### Immunization of Mice

BALB/c mice (Charles River Laboratories, Charles River, NY), 6-8 weeks old, were initially immunized subcutaneously and intraperitoneally with 50 µg of the HCV peptide to the putative envelope region p380-436, in 100 µl of Freund's complete adjuvant (Difco, Detroit, Ml). On day 15, 50 µg of the immunogen was diluted into 100 µl of phosphate buffered saline (PBS), pH 7.2, and injected intravenously into the tail vein (J. Goding, Monoclonal Antibodies: Principles and Practice [New York; Academic Press, 1986]). Sera titers were not evaluated.

### Fusion

On day 18, mice were sacrificed and splenocytes were fused in a 1:1 ratio with the SP2/0 myeloma line according to known conventional methods [G. Kohler and C. Milstein, Nature (1975) 256:495-497; J. Goding, supra]. The cell fusion pellet was dispersed with 1 ml 50% polyethylene glycol (PEG) (American Type Culture Collection, MW 1450) and centrifuged in Isoove's Modified Dulbecco's Medium (IMDM) (Gibco, Grand Island, NY). The cells were resuspended in HAT (hypoxanthine-aminoperin-thymidine)-selective IMDM with 10 % fetal bovine serum (FBS) (Hyclone Laboratories, Logan, UT) and plated at 3 x 10⁵ cells per 96-well tissue culture plates. Growth promoters included in the HAT media were 0.5% STM (RIBI Immunochem Research, Inc., Hamilton, MT) and 1% Origen Hybridoma Cloning Factor (Igen, Rockville, MD). Growth medium was replaced in culture wells post-fusion on day 5 and 7 using HT (hypoxanthine-thymidine) supplemented IMDM with 10% FBS.

### Enzyme Immunoassay (EIA)

Culture supemates were EIA screened 10 days post-fusion against the immunizing antigen to detect hybrids secreting HCV specific antibody and a non-specific protein to eliminate any false positives (Langone & Van Vunakis. eds., Methods in Enzymology, 92:168-174, Academic Press [1983]). Polystyrene 96-well microtiter plates were coated ovemight at room temperature with 50 µl per well of a 1 µg/ml of HCV peptide a.a. 380-436 in PBS. Any remaining binding sites on the polystyrene wells were blocked with 3% bovine serum albumin (BSA) (Intergen, Purchase, NY) in PBS for 30 minutes at room temperature. Plates were washed three times with distilled water. Fifty microliters of hybridoma tissue culture supematants were incubated for 1 hour at room temperature in the wells, and the wells were washed three times with distilled water. Antibody binding to antigen was detected using goat anti-mouse IgG+M-horseradish peroxidase (HRPO) (Kirkegaard-Perry Laboratories [KPL], Gaithersburg, MD) diluted at a concentration of 1:1000 in the block solution and incubated 30 minutes at room temperature. The plates were washed with distilled water and o-phenylenediamine substrate (OPD; Abbott Laboratories, Abbott Park, IL) was used as the chromogen. Plates were read at 492 nm. Hybrid cultures were regarded as potential HCV antibody-positive when the optical density (OD) was 3 times the negative control (NC) and significant preferential to the HCV antigen plate was observed compared to antibody binding of the irrelevant antigen coated plate, ie: >0.2 OD difference and <0.2 OD signal on the latter.

### Western Blot

Hybrid antibody specificity was confirmed with Westem blot analysis (Towbin & Gordon, J. Immunol. Methods. 72: 313-340 [1984]). HCV recombinant proteins and irrelevant proteins were electrophoresed by sodium dodecyl sulfate-polyacrylamide gel electrophoresis (SDS-PAGE) and then transferred to nitrocellulose, according to the manufacturers instructions (Schleicher & Schuell, Keene, NH; Bio-Rad, Richmond, CA). The nitrocellulose strips were blocked with 1% bovine hemoglobin (Sigma Chemical Co., St. Louis, MO) and 0.5% Tween-20 (Fisher Scientific, Pittsburgh, PA) in PBS for 30 minutes at room temperature, then the strips were incubated with hybrid tissue culture supernatant. The strips were then washed in PBS and goat anti-mouse IgG+M-HRPO (KPL) added for 30 minutes. Antibody binding to the HCV antigen was visualized with 4-chloro-1-naphthol (Sigma) as the chromogenic substrate. Hybrid cultures were cloned and placed in cryostorage if HCV antibody specificity was demonstrated.

### Establishment of Clones

HCV specific hybrids were cloned by limiting dilution (Goding, Monoclonal Antibodies: Principles and Practices, 2nd ed, Academic Press, New York [1986]). Modifications included plating of the cultures in log₁₀ dilution series and selecting positive clones for expansion from plates which exhibited <20% growth per 96 well tissue culture plate. Culture supemates were tested after 10 days using the EIA and Westem blot procedures described above. The selected clones were expanded for further evaluation and cryostored in 80% IMDM with 10% FBS (Hyclone) and 10% DMSO (Sigma).

### Monoclonal Antibody Isotype

Monoclonal antibody isotype was determined with the SBA Clonotyping System II kit (Southern Biotechnology Associates, Inc., Birmingham, AL) with modifications. EIA 96-well microtiter plates were coated ovemight at room temperature with 100 µl/well of a 1:1000 dilution of goat anti-mouse IgG+M (H+L) (KPL). Plates were blocked for 30 minutes with 3% BSA in PBS and washed with water. Culture samples were added to the wells, incubated for 1 hour, and washed with water. The kit's goat anti-mouse subtype specific conjugates were added for a 30 minute incubation period. Following a water wash, color was identified with OPD substrate. The goat anti-mouse isotype specific conjugate that bound to the mouse immunoglobin and displayed a >0.1 OD at 492 nm signaled the subtype.

### Monoclonal Antibody Production

Clones selected for further evaluation were scaled up in tissue cuture T-flasks and 10⁶ cells were injected into the peritoneal cavity of pre-pristaned BALB/c mice (Charles River Biotechnical Services, Inc., Wilmington, MA) (see Hurrell, supra). The resulting ascites fluid was harvested 7-10 days after injection, centrifuged, and stored at -20°C. The IgG antibody was affinity purified on Protein A (Pharmacia-LKB Biotechnologies, Piscataway, NJ) utilizing the automated OROS purification system Model 100 (see Goding, supra, for basic principles). The IgM antibodies were purified by molecular sizing on a S-300 column (Pharmacia-LKB).

All the following characterization information was performed with purified monoclonal antibody.

### Isolectric Focusing (IEF)

A cell line quality control to ensure consistency of frozen lots included measuring the antibody pl point on an IEF gel apparatus (Bio-Rad) which separates proteins based on net charge. Briefly, a bis-acrylamide-riboflavin solution was applied to an acrylamide gel, exposed to fluorescent lighting for 1 hour, then stored overnight at 4°C. A 1 µg sample of monoclonal antibody and standards were overlayed on the gel and electrophoresed over a 1-2 hour period. Following a series of fixatives and washes, the gel was silver stained (Bio-Rad). The pl value of the monoclonal antibody was calculated by migratory distance through the gel and was directly compared to the protein standards' migratory distance of known pl values. The distinctive finger print banding pattem reflected the pl microheterogeneity between independently produced lots of antibody (Hamilton, R.G., Reimer, C.B., Rodkey, L.S. (1987) Quality control of murine monoclonal antibodies using isoelectric focusing affinity immunoblot analysis. Hybridoma 6:205-217).

### EIA and Western Blot Specificity of Monoclonal Antibodies

All monoclonal antibodies noted herein were screened on an assortment of available recombinant HCV antigens.

The procedures were as outlined above. The multiple antigen screening technique confirmed the HCV specificity and excluded the HCV non-spectfic CKS, λPL, or linker-arm reactivity of the monoclonal antibodies.

### EIA Epitope Competition Studies

To investigate specificity and antigen binding distinctions, epitope grouping experiments were performed utilizing biotin labeled and unlabeled monoclonal antibodies (Langone & Van Vunakis, Methods in Enzymology, 92:242-253, Academic Press [1983]). Briefly, the antibodies were labeled with NHS-LC-biotin (Pierce Chemical Co., Rockford, IL) according to the manufacturers instructions. Microtiter wells were coated with the immunogen as previously described. First, log₂ dilutions of the unlabeled antibody were pre-incubated in the wells for 15 minutes, followed by the addition of a fixed amount of biotinylated antibody (the dilution in a direct EIA of the biotinylated antibody alone which gave a value of 50% of the maximum absorbance value) and incubated for 20 minutes. Plates were washed three times with water. Diluted streptavidin-HRPO (Zymed, South San Francisco, CA) was added to the wells and incubated for 30 minutes. The plates were washed again and OPD color developed as previously described. The absorbance was read at 492 nm. Antibodies of the same or related epitope had signal blocked or inhibited by >50%. No inhibition was observe with antibodies of distinct specificity. This was performed reciprocally for antibodies produced with the ENV regions.

### Peptide Inhibition Assays

Synthetic peptides were synthesized as previously described for HCV amino acid sequences 385-436 and 405-436. These peptides were employed in competition assays according to the procedure previously described by substituting serial dilutions of the peptides in place of the unlabeled antibody. Fifty microliters of labeled antibody (50% maximun absorbance value) was pre-incubated with the peptides for 15 minutes in a separate 96-well tissue culture dish. Next, 50 ul of the peptide and labeled monoclonal antibody mixture was added to the previously blocked antigen coated EIA plate and incubated for 20 minutes. Streptavidin-HRPO goat anti-mouse conjugated (Zymed) was employed to dectect the immune complexes formed.

### RIA Reciprocal Competition

Beads coated with the appropriate antigen or peptide were incubated with 100 µl of unlabeled monoclonal antibody diluted into recalcified negative human plasma (NHP, testing negative for anti-HCV, anti-HIV and HBsAg) at monoclonal antibody concentrations of 1-20 µg/ml. 100 µl of radiolabeled antibody at 1 to 4 µCi/ml diluted into HTLV I kit specimen diluent (containing detergent, animal sera, buffer, available from Abbott Laboratories, Abbott Park, IL) was incubated with the bead for 2 hours at 45° or 18-20 hours at 20-25°C. Beads were washed and counted for radioactivity.

### HCV Antigen Assays

Beads coated with one or a cocktail of anti-HCV monoclonal antibodies were incubated with 200 µl of specimen for 2 hours at 40-45°C or 18-20 hours at 20-25°C. Beads were washed with distilled water and then incubated with 200 µl of radiolabeled anti-HCV monoclonal antibody (one or more) for 2 hours at 45°C. Beads were washed and counted in a gamma counter.

### Characterization of monoclonal antibody

Monoclonal antibodies against the HCV ENV domain (380-436) AND (405-436) are characterized in Tables 1 and 2. Referring to FIGS. 2 to 13, the reactivities summarized below in Table 1 are shown in lanes 7 and 8. Lanes 1 to 3 contains monoclonal antibodies against HCV 33C protein (6-296-534 in lane 1, 6-914-518 in lane 2 and 6-1070-110 in lane 3); lanes 4-6 contain monoclonal antibodies against HCV CORE (13-975-157 in lane 4, 14-153-234 in lane 5 and 14-1350-210 in lane 6); lanes 7 and 8 contain monoclonal antibodies against the putative HCV ENV region (16-407-209 in lane 7 and 16-803-174 in lane 8); lanes 9-11 contain monoclonal antibodies against HCV C-100 (25-1518-105 in lane 9, 28-735-355 in lane 10; CKS control monoclonal antibody 29-121-236 in lane 11); lane 12 contains a normal mouse serum control; and lane 13 contains a negative control.

**TABLE 1**

| Reactivity on Western Blot | | | | | | | | | | | | |
|---|---|---|---|---|---|---|---|---|---|---|---|---|
| Cell Line | CKS-core | λPL-core | λPL-c33-core | CKS-c33 | CKS-c33-BCD | CKS-BCD | CKS-B | CKS-E | CKS- | SOD 100 | CKS-A'BCD | CKS-A"BCD |
| 16-407-209 | - | - | - | - | - | - | - | - | - | - | - | - |
| 16-803-174 | - | - | - | - | - | - | - | - | - | - | - | - |

**TABLE 2.**

| Reactivity on ElA | | | | | | | |
|---|---|---|---|---|---|---|---|
| Groupe# | Cell Line | pl | Isotype | ENV 380-436 | ENV 405-436 | λPL 33c-core | CKS-33c |
| 1 | 16-407-209 | 7.0 | IgG3 k | + | + | - | - |
| 2 | 16-803-174 | | IgMk k | + | + | - | - |

The following examples demonstrate the advantages and utility of this invention for serodiagnosis of HCV by describing methods for the clinical utility of these monoclonal antibodies.

### EXAMPLES

### Example 1

### Anti-HCV ENV Competitive Assay

Ten (10) specimens from blood bank donors with elevated alanine aminotransferase (ALT) were tested in a competitive one-step assay described hereinabove for "RIA Reciprocal Competition" for the detection of anti-HCV ENV. The data are presented in Table 3. Referring to Table 3, if >25% inhibition is considered reactive in this assay, then one of ten human test samples with elevated ALT was reactive for anti-HCV ENV.

**TABLE 3**

| Anti-ENV Immunoassay | | | | | | | | | | |
|---|---|---|---|---|---|---|---|---|---|---|
| Bead: 380 peptide (p380-436) | | | | | | | | | | |
| Specimen | | Label: 16-958 | | | | Label 16-407 | | | | |
| | CPM | AVG | S/N | %Inhib | Result | CPM | AVG | S/N | %Inhib | Result |
| NC | 1535 | 1621 | | | | 1032 | 1015 | | | |
| | 1779 | | | | | 977 | | | | |
| | 1550 | | | | | 1036 | | | | |
| | | | | | | | | | | |
| 27 | 2862 | 2571 | 1.59 | -58.605799 | - | 1079 | 1136 | 1.12 | -11.8719 | - |
| | 2280 | | | | | 1192 | | | | |
| | | | | | | | | | | |
| 238 | 1295 | 1295 | 0.75 | 24.9228871 | + | 767 | 798 | 0.79 | 21.42857 | + |
| | 1139 | | | | | 828 | | | | |
| | | | | | | | | | | |
| 135 | 1606 | 2054 | 1.27 | -26.711906 | - | 869 | 852 | 0.84 | 16.10837 | - |
| | 2502 | | | | | 834 | | | | |
| 163 | 3128 | 3058 | 1.89 | -88.648982 | - | 1377 | 1464 | 1.44 | -44.2365 | - |
| | 2988 | | | | | 1551 | | | | |
| | | | | | | | | | | |
| 173 | 2240 | 2154 | 1.33 | -32.850093 | - | 1128 | 1034 | 1.02 | -1.82266 | - |
| | 2067 | | | | | 939 | | | | |
| | | | | | | | | | | |
| 220 | 3503 | 3232 | 1.99 | -99.352252 | - | 1119 | 1065 | 1.05 | -4.87685 | - |
| | 2960 | | | | | 1010 | | | | |
| | | | | | | | | | | |
| 252 | 4208 | 4149 | 2.56 | -155.92227 | - | 1222 | 1150 | 1.13 | -13.5005 | - |
| | 4098 | | | | | 1078 | | | | |
| | | | | | | | | | | |
| 283 | 3330 | 3197 | 1.97 | -97.193091 | - | 1059 | 1039 | 1.02 | -2.31527 | - |
| | 3063 | | | | | 1018 | | | | |
| | | | | | | | | | | |
| 28 | 4014 | 3829 | 2.36 | -136.21221 | - | 1215 | 1306 | 1.29 | -28.67 | - |
| | 3644 | | | | | 1397 | | | | |
| | | | | | | | | | | |
| 290 | 3701 | 3542 | 2.19 | -118.50709 | - | 1314 | 1290 | 1.27 | -27.0443 | - |
| | 3883 | | | | | 1265 | | | | |
| | | | | | | | | | | |
| 16-803-174 | 41 | 46 | 0.03 | 97.1622455 | + | 220 | 259 | 0.26 | 74.48276 | + |
| | 51 | | | | | 298 | | | | |
| | | | | | | | | | | |
| PC813 | 1858 | 1876 | 1.16 | -15.73103 | - | 1065 | 1138 | 1.12 | -12.1511 | - |
| | 1878 | | | | | 1088 | | | | |
| | 1892 | | | | | 1262 | | | | |

### Example 2

### HCV ENV Antigen Assay

Antigen assays were conducted using the HCV Antigen Assay described herein as "HCV Antigen Assays." Results from a two step ENV antigen assay are shown in Table 4. The most sensitive assay for detection of 380-436 ENV synthetic peptide was a bead coated with monoclonal antibodies 16-407-209 and 16-803-174 label. The sensitivity for synthetic peptide was less than 10 µg/ml.

**TABLE 4**

| HCV ENV Antigen Assay | | | | | | | | |
|---|---|---|---|---|---|---|---|---|
| | (16-407-209 Bead: 16-803-174 Label) | | | | (16-803-174 Bead:16-407-209 Label) | | | |
| Specimen | CPM | AVG | S/N | Result | CPM | AVG | S/N | Result |
| NC | 205 | 206 | | | 230 | 221 | | |
| | 213 | | | | 235 | | | |
| | 199 | | | | 198 | | | |
| NC + 20%NaAc | 228 | 229 | 1.11 | - | 256 | 256 | 1.16 | - |
| | | 229 | | | | 256 | | |
| 380-436) | 1537 | 1470 | 7.13 | + | 746 | 709 | 3.21 | + |
| (20 µg/ml | 1402 | | | | 672 | | | |
| | | | | | | | | |
| 380-436 | 2685 | 2575 | 12.50 | + | 646 | 660 | 2.98 | + |
| (200 µg/ml) | 2464 | | | | 673 | | | |

### Example 3

### HCV Antibody Test Employing Cocktails of Monoclonal Antibodies

Antigen assays were conducted using the HCV Antigen Assay described herein as "HCV Antigen Assays." Another variation of this assay used a cocktail of monoclonal antibody on the bead (16-407, 16-803, and 16-1291) and a cocktail label (16-407 and 16-803). It was found that one specimen of 36 obtained from the Interstate Blood Bank (designated as number 13) showed significant reactivity in this assay.

Thus, the novel monoclonal antibodies of the invention can be used in a variety of ways. These monoclonal antibodies can be used for immunoprecipitation of amplified product and detection of HCV nucleic acid microparticles or carrier coated with anti-HCV monoclonal antibody used to capture virus or viral protein associated with HCV RNA. Then detection methodology for RNA may be used.

These monoclonal antibodies also can be used for localization of HCV antigens within the cell using HCV monoclonal antibody tagged directly (fluorescence, colloidal gold, etc.) or using secondary tagged anti-mouse antibody. Histopathology of disease may be tracked. Further, the detection of native or recombinant HCV antigens in sera, tissue, cells, culture media, or body fluid using individual monoclonal antibodies in a sandwich configuration or a cocktail of monoclonal antibodies on the solid phase and in the detection system.

One step antigen assays using monoclonal antibodies against non overlapping epitopes may also be performed. Some monoclonal antibodies may recognize antigenic epitopes not recognized by the infected individual and therefore may be possible to recognize serum Ag both free and bound with human antibody. Furthermore, "cryptic" or hidden antigens or antigenic determinants may be uncovered by treatment of specimen with detergent or reducing agent or both. For example, CORE antigen may exist in a capsid form covered by the virus envelope. Stripping the envelope with detergent should expose CORE antigen. Monoclonal antibodies may also offer pragmatic advantages over high titer polyclonal antibody in giving greater sensitivity in assay or allowing shorter incubation times.

Further, antibody immunoassays, one or two step competitive assays, were developed in which anti-HCV competed with labeled anti-HCV monoclonal antibody for binding to a limited number of antigenic sites. A more sensitive competitive assay may be developed in which human anti-HCV binds to HCV Ag in solution blocking or inhibiting the HCV Ag binding in HCV Ag sandwich assay. Competitive assays using monoclonal antibodies allow a more precise mapping of human antibody epitopes and may be useful for determining virus neutralizing antibody epitopes. Some monoclonal antibodies may have virus neutralizing activity. Finally, monoclonal antibodies should be useful in immunoaffinity purification of native viral and recombinant HCV antigens and proteins.

The hybridoma cell lines which secrete the monoclonal antibodies of the invention are identified as hybridoma cell line 16-407-209 (secreting monoclonal antibody 16-407-209) and hybridoma cell line 16-803-174 (secreting hybridoma cell line 16-803-174). These hybridoma cell lines were deposited at the American Type Culture Collection, 12301 Parklawn Drive, Rockville, Maryland 20852 on November 16, 1990 under the terms of the Budapest Treaty and were accorded the following deposit numbers: Hybridoma cell line 16-407-209 was accorded A.T.C.C. deposit No. HB 10601, and hybridoma cell line 16-803-174 was accorded A.T.C.C. deposit No. HB 10605.

Other variations of applications of the use of the unique monoclonal antibodies provided herein include the detection of HCV antigen in immune complexes, or latent and/or cryptic antigens, and/or associated with viral nucleic acid for detection of the nucleic acid by PCR, LCR, or by direct hybridization.

## Claims

1. A monoclonal antibody or fragment thereof which specifically binds to HCV envelope region and has the binding specificity of the monoclonal antibody secreted by hybridoma cell line HB 10601.

2. A monoclonal antibody or fragment thereof which specifically binds to HCV envelope region and has the binding specificity of the monoclonal antibody secreted by hybridoma cell line HB 10605.

3. A monoclonal antibody secreted by A.T.C.C. deposit No. HB 10601.

4. A monoclonal antibody secreted by A.T.C.C. deposit No. HB 10605.

5. A hybridoma cell line which secretes a monoclonal antibody which specifically binds to HCV envelope region, said hybridoma cell line having all of the identifying characteristics of hybridoma cell line A.T.C.C. deposit No. HB 10601.

6. A hybridoma cell line which secretes a monoclonal antibody which specifically binds to HCV envelope region, said hybridoma cell line having all of the identifying characteristics of hybridoma cell line A.T.C.C. deposit No. HB 10605.

7. A hybridoma cell line A.T.C.C. deposit No. HB 10601.

8. A hybridoma cell line A.T.C.C. deposit No. HB 10605.

9. A sandwich assay method for determining the presence of HCV in a test sample which may contain HCV, comprising:
a. contacting the test sample with at least an anti-HCV envelope region antibody attached to a solid phase which antibody specifically binds to HCV envelope region, to form a mixture, said antibody being a monoclonal antibody, or fragment thereof, or a combination of monoclonal antibodies, said monoclonal antibodies having the binding specificity of the monoclonal antibody secreted by the hybridoma A.T.C.C. deposit No. HB 10601 or A.T.C.C. deposit No. HB 10605;
b. incubating said mixture for a time and under conditions sufficient to form antigen/antibody complexes;
c. contacting said complexes with an indicator reagent comprising a signal generating compound capable of generating a measurable detectable signal attached to an anti-HCV envelope region antibody, to form a second mixture, said antibody being a monoclonal antibody, or fragment thereof, or a combination of monoclonal antibodies, said monoclonal antibodies having the binding specificity of the monoclonal antibody secreted by the hybridoma A.T.C.C. deposit No. HB 10601 or A.T.C.C. deposit No. HB 10605;
d. incubating said second mixture for a time and under conditions sufficient to form antibody/antigen/antibody complexes; and
e. determining the presence of HCV in the test sample by detecting the measurable signal generated.

10. The method of claim 9 wherein the amount of HCV present in the test sample is proportional to said measurable signal.

11. The method of claim 10 wherein the signal generating compound is selected from the group consisting of a luminescent compound, a chemiluminescent compound, an enzyme and a radioactive element.

12. The method of claim 11 wherein said enzyme is selected from the group consisting of horseradish peroxidase, alkaline phosphatase and beta-galactosidase.

13. The method of claim 12 wherein said enzyme is horseradish peroxidase.

14. A sandwich assay method for determining the presence and amount of HCV which may be present in a test sample, comprising:
a. contacting a test sample with a monoclonal anti-HCV envelope region antibody attached to a solid phase and an indicator reagent comprising a monoclonal antibody which specifically binds to HCV envelope region attached to a signal generating compound capable of generating a measurable detectable signal to form a mixture, said antibodies being monoclonal antibodies, or fragment thereof, or a combination of monoclonal antibodies, said monoclonal antibodies having the binding specificities of the monoclonal antibodies secreted by the hybridoma A.T.C.C. deposit No. HB 10601 or A.T.C.C. deposit No. HB 10605;
b. incubating said mixture for a time and under conditions sufficient to form antibody/antigen/antibody complexes;
c. determining the presence of HCV present in the test sample by detecting the measurable signal as an indication of the presence of HCV in the test sample.

15. The method of claim 14 wherein the amount of HCV present in the test sample is proportional to the measurable signal generated.

16. A competitive assay method for determining the presence and amount of HCV antibody which may be present in a test sample, comprising:
a. contacting a test sample suspected of containing HCV antibodies with an indicator reagent comprising a signal generating compound and a monoclonal antibody which specifically binds to HCV envelope proteins and a solid phase coated with HCV envelope proteins, for a time and under conditions sufficient to form antigen/antibody complexes of the test sample and solid phase and/or indicator reagent and solid phase, said monoclonal antibody, or fragment thereof, or a combination of said monoclonal antibodies having the binding specificity of the monoclonal antibody secreted by the hybridoma A.T.C.C. deposit No. HB 10601 or A.T.C.C. deposit No. HB 10605;
b. determining the presence of HCV antibody present in the test sample by detecting the reduction in binding of the indicator reagent to the solid phase as compared to the signal generated from a negative test sample to indicate the presence of HCV antibody in the test sample.

17. The method of claim 16 wherein the signal generating compound is selected from the group consisting of a luminescent compound, a chemiluminescent compound, an enzyme and a radioactive element.

18. The method of claim 17 wherein said enzyme is selected from the group consisting of horseradish peroxidase, alkaline phosphatase and beta-galactosidase.

19. The method of claim 18 wherein said enzyme is horseradish peroxidase.

20. An assay kit for determining the presence of HCV in a test sample comprising:
a container containing at least one monoclonal antibody, or fragment thereof, which specifically binds to HCV envelope region wherein said monoclonal antibody has the binding specificity of the monoclonal antibody secreted by the cell line A.T.C.C. deposit No. HB 10601 or A.T.C.C. deposit No. HB 10605.

## Patentansprüche

1. Ein monoklonaler Antikörper oder Fragment davon, der spezifisch an den HCV Hüllbereich bindet und der die Bindungsspezifität des monoklonalen Antikörpers aufweist, der von der Hybridomzellinie HB 10601 abgesondert wird.

2. Ein monoklonaler Antikörper oder Fragment davon, der spezifisch an den HCV Hüllbereich bindet und der die Bindungsspezifität des monoklonalen Antikörpers aufweist, der von der Hybridomzellinie HB 10605 abgesondert wird.

3. Monoklonaler Antikörper, der von A.T.C.C. Hinterlegungsnummer HB 10601 abgesondert wird.

4. Monoklonaler Antikörper, der von A.T.C.C. Hinterlegungsnummer HB 10605 abgesondert wird.

5. Hybridomzellinie, die einen monoklonalen Antikörper absondert, der spezifisch an den HCV Hüllbereich bindet, wobei die Hybridomzellinie alle kennzeichnenden Eigenschaften der Hybridomzellinie A.T.C.C. mit der Hinterlegungsnummer HB 10601 aufweist.

6. Hybridomzellinie, die einen monoklonalen Antikörper absondert, der spezifisch an den HCV Hüllbereich bindet, wobei die Hybridomzellinie alle kennzeichnenden Eigenschaften der Hybridomzellinie A.T.C.C. mit der Hinterlegungsnummer HB 10605 aufweist.

7. Hybridomzellinie A.T.C.C. Hinterlegungsnummer HB 10601.

8. Hybridomzellinie A.T.C.C. Hinterlegungsnummer HB 10605.

9. Sandwichassayverfahren zur Bestimmung der Anwesenheit von HCV in einer Testprobe, die HCV enthalten kann, die folgendes umfaßt:
a. In-Kontakt-Bringen der Testprobe, unter Ausbildung einer Mischung, mit wenigstens einem anti-HCV Hüllregion-Antikörper, der an eine feste Phase gebunden ist, wobei der Antikörper spezifisch an die HCV Hüllregion bindet, und wobei der Antikörper ein monoklonaler Antikörper ist oder ein Fragment davon, oder eine Kombination von monoklonalen Antikörpern, wobei die monoklonalen Antikörper die Bindungsspezifität des monoklonalen Antikörpers aufweisen, der vom Hybridom A.T.C.C. Hinterlegungsnummer HB 10601 oder A.T.C.C. Hinterlegungsnummer HB 10605 abgesondert wird;
b. Inkubation der Mischung einen Zeitraum lang und unter Bedingungen, die zur Ausbildung von Antigen/Antikörper-Komplexen hinreichend sind;
c. In-Kontakt-Bringen der Komplexe mit einem Indikatorreagens, das eine signalerzeugende Verbindung umfaßt, die zur Erzeugung eines meßbaren nachweisbaren Signals befähigt ist, die an einen anti-HCV-Hüllregion- Antikörper angebunden ist, unter Ausbildung einer zweiten Mischung, wobei der Antikörper ein monoklonaler Antikörper oder ein Fragment davon ist, oder eine Kombination von monoklonalen Antikörpern, wobei die monoklonalen Antikörper die Bindungsspezifität des monoklonalen Antikörpers aufweisen, der von dem Hybridom A.T.C.C. Hinterlegungsnummer HB 10601 oder A.T.C.C. Hinterlegungsnummer 10605 abgesondert wird;
d. Inkubation der zweiten Mischung eine Zeit lang und unter Bedingungen, die zur Ausbildung von Antikörper/Antigen/Antikörper-Komplexen hinreichend sind; und
e. Bestimmung der Anwesenheit von HCV in der- Testprobe durch den Nachweis des erzeugten meßbaren Signals.

10. Verfahren nach Anspruch 9, worin die Menge an HCV, die in der Testprobe vorhanden ist, zum meßbaren Signal proportional ist.

11. Verfahren nach Anspruch 10, worin die signalerzeugende Verbindung aus der Gruppe gewählt ist, die aus einer lumineszenten Verbindung, einer chemilumineszenten Verbindung, einem Enzym und einem radioaktiven Element gewählt ist.

12. Verfahren nach Anspruch 11, worin das Enzym aus der Gruppe gewählt ist, die aus Meerrettichperoxidase, alkalischer Phosphatase und beta-Galaktosidase besteht.

13. Verfahren nach Anspruch 12, worin das Enzym Meerrettichperoxidase ist.

14. Sandwichassayverfahren zur Bestimmung der Anwesenheit und Menge von HCV, die in einer Testprobe vorhanden sein kann, das folgendes umfaßt:
a. In-Kontakt-Bringen einer Testprobe mit einem monoklonalen anti-HCV-Hüllregion-Antikörper, der an eine feste Phase gebunden ist und mit einem Indikatorreagenz, das einen monoklonalen Antikörper umfaßt, der spezifisch an die HCV-Hüllregion bindet, der an eine signalerzeugende Verbindung gebunden ist, die ein meßbares nachweisbares Signal zu erzeugen vermag, unter Ausbildung einer Mischung, wobei die Antikörper monoklonale Antikörper oder Fragmente davon oder eine Kombination von monoklonalen Antikörpern sind, wobei die monoklonalen Antikörper die Bindungsspezifitäten der monoklonalen Antikörper aufweisen, die von dem Hybridom A.T.C.C. Hinterlegungsnummer HB 10601 oder A.T.C.C. Hinterlegungsnummer 10605 abgesondert werden;
b. Inkubation der Mischung eine Zeit lang -und unter Bedingungen, die für die Ausbildung von Antikörper/Antigen/Antikörper-Komplexen hinreichend sind;
c. Bestimmung der Anwesenheit von HCV, das in der Testprobe vorhanden ist durch den Nachweis des meßbaren Signals als Anzeige für die Anwesenheit von HCV in der Testprobe.

15. Verfahren nach Anspruch 14, worin die Menge an in der Testprobe vorhandenem HCV zum erzeugten meßbaren Signal proportional ist.

16. Kompetitivassayverfahren zur Bestimmung der Anwesenheit und Menge an HCV Antikörper, der in einer Testprobe vorhanden sein kann, das folgendes umfaßt:
a. In-Kontakt-Bringen einer Testprobe, von der vermutet wird, daß sie HCV Antikörper enthält, mit einem Indikatorreagenz, das eine signalerzeugende Verbindung und einen monoklonalen Antikörper umfaßt, der spezifisch an HCV Hüllproteine bindet und mit einer festen Phase, die mit HCV Hüllproteinen beschichtet ist, eine Zeit lang und unter Bedingungen, die zur Ausbildung von Antigen/Antikörper-Komplexen aus der Testprobe und der festen Phase und/oder dem Indikatorreagenz und der festen Phase hinreichend sind, wobei der monoklonale Antikörper, oder ein Fragment davon, oder eine Kombination der monoklonalen Antikörper die Bindungsspezifität des monoklonalen Antikörpers aufeist/aufweisen, der von dem Hybridom A.T.C.C. Hinterlegungsnummer 10601 oder A.T.C.C. Hinterlegungsnummer HB 10605 abgesondert wird;
b. Bestimmung der Anwesenheit von HCV Antikörper, der in der Testprobe vorhanden ist, durch den Nachweis des Rückgangs der Bindung des Indikatorreagenzes an die feste Phase verglichen mit dem Signal, das von einer negativen Testprobe erzeugt wird, um die Anwesenheit von HCV Antikörper in der Testprobe anzuzeigen.

17. Verfahren nach Anspruch 16, worin die signalerzeugende Verbindung aus der Gruppe gewählt ist, die aus einer lumineszenten Verbindung, einer chemilumineszenten Verbindung, einem Enzym und einem radioaktiven Element besteht.

18. Verfahren nach Anspruch 17, worin das Enzym aus der Gruppe gewählt ist, die aus Meerrettichperoxidase, alkalischer Phosphatase und beta-Galaktosidase besteht.

19. Verfahren nach Anspruch 18, worin das Enzym Meerrettichperoxidase ist.

20. Assaykit zur Bestimmung der Anwesenheit von HCV in einer Testprobe, das folgendes umfaßt:
einen Behälter, der wenigstens einen monoklonalen Antikörper oder ein Fragment davon enthält, der/das spezifisch an die HCV Hüllregion bindet, wobei der monoklonale Antikörper die Bindungsspezifität des monoklonalen Antikörpers aufweist, der von der Zellinie A.T.C.C. Hinterlegungsnummer HB 10601 oder A.T.C.C. Hinterlegungsnummer HB 10605 abgesondert wird.

## Revendications

1. Anticorps monoclonal ou fragment de celui-ci qui se lie spécifiquement à la région d'enveloppe de HCV et qui a la spécificité de liaison de l'anticorps monoclonal sécrété par la lignée cellulaire d'hybridome n° HB 10601.

2. Anticorps monoclonal ou fragment de celui-ci qui se lie spécifiquement à la région d'enveloppe de HCV et qui la spécificité de liaison de l'anticorps monoclonal sécrété par la lignée cellulaire d'hybridome n° HB 10605.

3. Anticorps monoclonal sécrété par le dépôt A.T.C.C. n° HB 10601.

4. Anticorps monoclonal sécrété par le dépôt A.T.C.C. n° HB 10605.

5. Lignée cellulaire d'hybridome qui sécrète un anticorps monoclonal qui se lie spécifiquement à la région d'enveloppe de HCV, ladite lignée cellulaire d'hybridome ayant toutes les caractéristiques d'identification de la lignée cellulaire d'hybridome n° de dépôt A.T.C.C. HB 10601.

6. Lignée cellulaire d'hybridome qui sécrète un anticorps monoclonal qui se lie spécifiquement à la région d'enveloppe de HCV, ladite lignée cellulaire d'hybridome ayant toutes les caractéristiques d'identification de la lignée cellulaire d'hybridome n° de dépôt A.T.C.C. HB 10605.

7. Lignée cellulaire d'hybridome n° de dépôt A.T.C.C. HB 10601.

8. Lignée cellulaire d'hybridome n° de dépôt A.T.C.C. HB 10605.

9. Procédé de dosage en sandwich pour déterminer la présence de HCV dans un échantillon-test qui peut contenir HCV, comprenant :
a. la mise en contact de l'échantillon-test avec au moins un anticorps anti-région d'enveloppe de HCV fixé à une phase solide, lequel anticorps se lie spécifiquement à la région d'enveloppe de HCV, pour former un mélange, ledit anticorps étant un anticorps monoclonal, ou un fragment de celui-ci, ou une combinaison d'anticorps monoclonaux, lesdits anticorps monoclonaux ayant la spécificité de liaison de l'anticorps monoclonal sécrété par l'hybridome n° de dépôt A.T.C.C. MB 10601 ou n° de dépôt A.T.C.C. MB 10605;
b. l'incubation dudit mélange pendant une durée et dans des conditions suffisantes pour former des complexes antigène/anticorps;
c. la mise en contact desdits complexes avec un réactif indicateur comprenant un composé générateur de signal capable de produire un signal détectable mesurable fixé à un anticorps anti-région d'enveloppe de HCV, pour former un second mélange, ledit anticorps étant un anticorps monoclonal, ou un fragment de celui-ci, ou une combinaison d'anticorps monoclonaux, lesdits anticorps monoclonaux ayant la spécificité de liaison de l'anticorps monoclonal sécrété par l'hybridome n° de dépôt A.T.C.C. HB 10601 ou n° de dépôt A.T.C.C. HB10605 ;
d. l'incubation dudit second mélange pendant une durée et dans des conditions suffisantes pour former des complexe anticorps/antigène/anticorps ; et
e. la détermination de la présence de HCV dans l'échantillon-test par détection du signal mesurable produit.

10. Procédé selon la revendication 9, où la quantité de HCV présente dans l'échantillon-test est proportionnelle audit signal mesurable.

11. Procédé selon la revendication 10, où le composé générateur de signal est choisi dans le groupe consistant en un composé luminescent, un composé chimiluminescent, une enzyme ou un élément radioactif.

12. Procédé selon la revendication 11, où ladite enzyme est choisie dans le groupe consistant en la peroxydase de raifort, la phosphatase alcaline et la bêta-galactosidase.

13. Procédé selon la revendication 12, où ladite enzyme est la peroxydase de raifort.

14. Procédé de dosage en sandwich pour déterminer la présence et la quantité de HCV qui peut être présent dans un échantillon-test, comprenant :
a. la mise en contact d'un échantillon-test avec un anticorps monoclonal anti-région d'enveloppe de HCV fixé à une phase solide et avec un réactif indicateur comprenant un anticorps monoclonal qui se lic spécifiquement à la région d'enveloppe de HCV fixé à un composé générateur de signal capable de produire un signal détectable mesurable pour former un mélange, lesdits anticorps étant des anticorps monoclonaux, ou un fragment de ceux-ci, ou une combinaison d'anticorps monoclonaux, lesdits anticorps monoclonaux ayant les spécificités de liaison des anticorps monoclonaux sécrétés par l'hybridome n° de dépôt A.T.C.C. HB 10601 ou n° de dépôt A.T.C.C. HB 10605 ;
b. l'incubation dudit mélange pendant une durée et dans des conditions suffisantes pour former des complexes anticorps/antigènes/anticorps ;
c. la détermination de la présence de HCV présent dans l'échantillon-test par détection du signal mesurable en tant qu'indication de la présence de HCV dans l'échantillon-test.

15. Procédé selon la revendication 14, où la quantité de HCV présente dans l'échantillon-test est proportionnelle au signal mesurable produit.

16. Procédé de dosage compétitif pour déterminer la présence et la quantité d'anticorps anti-HCV qui peut être présent dans l'échantillon-test, comprenant :
a. la mise en contact d'un échantillon-test supposé contenir des anticorps anti-HCV avec un réactif indicateur comprenant un composé générateur de signal et un anticorps monoclonal qui se lie spécifiquement à des protéines d'enveloppe de HCV et une phase solide recouverte de protéines d'enveloppe de HCV, pendant une durée et dans des conditions suffisantes pour former des complexes antigène/anticorps de l'échantillon-test et de la phase solide et/ou du réactif indicateur et de la phase solide, ledit anticorps monoclonal, ou un fragment de celui-ci, ou une combinaison desdits anticorps monoclonaux ayant la spécificité de liaison de l'anticorps monoclonal sécrété par l'hybridome n° de dépôt A.T.C.C. HB 10601 ou n° de dépôt A.T.C.C. HB 10605 ;
b. la détermination de la présence d'un anticorps anti-HCV présent dans l'échantillon-test par détection de la réduction de la liaison du réactif indicateur à la phase solide par rapport au signal produit par un échantillon test négatif pour indiquer la présence d'un anticorps anti-HCV dans l'échantillon-test.

17. Procédé selon la revendication 16, où le composé générateur de signal est choisi dans le groupe consistant en un composé luminescent, un composé chimiluminescent, une enzyme et un élément radioactif.

18. Procédé selon la revendication 17, où ladite enzyme est choisie dans le groupe consistant en la peroxydase de raifort, la phosphatase alcaline et la bêta-galactosidase.

19. Procédé selon la revendication 18, où ladite enzyme est la peroxydase de raifort.

20. Trousse de dosage pour déterminer la présence de HCV dans un échantillon-test comprenant :
un récipient contenant au moins un anticorps monoclonal, ou un fragment de celui-ci, qui se lie spécifiquement à la région d'enveloppe de HCV, ledit anticorps monoclonal ayant la spécificité de liaison de l'anticorps monoclonal sécrété par la lignée cellulaire n° de dépôt A.T.C.C. HB 10601 ou n° de dépôt A.T.C.C. HB 10605.
